(19) Europäisches Patentamt European Patent Office Office européen des brevets

(11) **EP 3 535 595 B1**

(12) **EUROPEAN PATENT SPECIFICATION**

(45) Date of publication and mention of the grant of the patent:
**03.01.2024 Bulletin 2024/01**

(21) Application number: **17798271.7**

(22) Date of filing: **07.11.2017**

(51) International Patent Classification (IPC):
**G01R 33/50** $^{(2006.01)}$ **G01R 33/561** $^{(2006.01)}$
**A61B 5/055** $^{(2006.01)}$ **A61B 5/00** $^{(2006.01)}$

(52) Cooperative Patent Classification (CPC):
**G01R 33/50; A61B 5/055; A61B 5/7203; A61B 5/7235; G01R 33/5614**

(86) International application number:
**PCT/GB2017/053344**

(87) International publication number:
**WO 2018/083504 (11.05.2018 Gazette 2018/19)**

(54) **CORRECTION METHOD FOR MAGNETIC RESONANCE T1-MAPPING OF VISCERAL ORGANS IN THE PRESENCE OF ELEVATED IRON AND ELEVATED FAT LEVELS, AND IN THE PRESENCE OF OFF-RESONANCE FREQUENCIES**

KORREKTURVERFAHREN FÜR MAGNETRESONANZ-T1-KARTIERUNG VON VISZERALEN ORGANEN IN ANWESENHEIT VON ERHÖHTEN EISEN- UND ERHÖHTEN FETTKONZENTRATIONEN UND IN ANWESENHEIT VON FREQUENZEN AUSSERHALB DER RESONANZ

PROCÉDÉ DE CORRECTION POUR LE MAPPAGE T1 DE RÉSONANCE MAGNÉTIQUE D'ORGANES VISCÉRAUX EN PRÉSENCE DE TAUX DE FER ET DE GRAISSE ÉLEVÉS, ET EN PRÉSENCE DE FRÉQUENCES HORS RÉSONANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2016 GB 201618750**

(43) Date of publication of application:
**11.09.2019 Bulletin 2019/37**

(73) Proprietor: **Oxford University Innovation Limited Botley Oxford OX2 0JB (GB)**

(72) Inventors:
• **ROBSON, Matthew Oxford Oxfordshire OX3 9DU (GB)**
• **MOZES, Ferenc Oxford Oxfordshire OX3 9DU (GB)**

(74) Representative: **Dehns St. Bride's House 10 Salisbury Square London EC4Y 8JD (GB)**

(56) References cited:
**GB-A- 2 513 474    GB-A- 2 524 587**

• **ELIZABETH M. TUNNICLIFFE ET AL: "A model for hepatic fibrosis: the competing effects of cell loss and iron on shortened modified Look-Locker inversion recovery T 1 (shMOLLI-T 1 ) in the liver : Hepatic Fibrosis and Iron in shMOLLI- T 1", JOURNAL OF MAGNETIC RESONANCE IMAGING, vol. 45, no. 2, 26 July 2016 (2016-07-26), pages 450-462, XP055459455, US ISSN: 1053-1807, DOI: 10.1002/jmri.25392**

**(Cont. next page)**

- **FERENC E. MOZES ET AL: "Influence of fat on liver T 1 measurements using modified Look-Locker inversion recovery (MOLLI) methods at 3T : Influence of Fat on Liver T 1 Measurements", JOURNAL OF MAGNETIC RESONANCE IMAGING, vol. 44, no. 1, 1 July 2016 (2016-07-01), pages 105-111, XP055458546, US ISSN: 1053-1807, DOI: 10.1002/jmri.25146**
- **DANIEL R. MESSROGHLI ET AL: "Modified Look-Locker inversion recovery (MOLLI) for high-resolution T1 mapping of the heart", MAGNETIC RESONANCE IN MEDICINE., vol. 52, no. 1, 28 June 2004 (2004-06-28), pages 141-146, XP055379973, US ISSN: 0740-3194, DOI: 10.1002/mrm.20110**
- **INTERNATIONAL SOCIETY FOR MAGNETIC RESONANCE IN MEDICINE, ISMRM, 2030 ADDISON STREET, 7TH FLOOR, BERKELEY, CA 94704 USA, no. 367, 7 April 2017 (2017-04-07), XP040687935,**

**Description**

The present disclosure generally relates to medical imaging and, more particularly, relates to systems and methods for performing processing of magnetic resonance (MR) imaging of the liver and other visceral organs which may be useful, for example, in making measurements relating to inflammation and fibrosis in the liver and other visceral organs.

As many as one in ten adults in the UK have some form of liver disease (British Liver Trust. Alcohol and liver disease. Ringwood: British Liver Trust, 2006). Liver disease is currently the fifth most common cause of mortality for both men and women (Department of Health. Quality Strategy Team Report on Liver Disease: A scoping study into the nature and burden of the disease, 2006). However, whilst the mortality rates for the other four major causes of death are falling, the trend for liver disease is rising in both sexes at an alarming rate and there has been a five-fold increase in the prevalence of liver cirrhosis in the last 30 years. The current childhood obesity epidemic, increasing alcohol misuse and viral hepatitis are all contributing to this.

The problem with liver disease is that often symptoms of the disease are not apparent until the disease reaches an advanced stage. Thus, there is a pressing need for a reliable diagnostic tool for liver disease to identify early disease and to target therapies to those patients that may benefit (e.g. antiviral therapy in progressive hepatitis C, weight reduction surgery in fatty liver disease).

The current accepted practice, or "gold standard", for diagnosing liver disease is an ultrasound-guided liver biopsy. This is less than ideal as there is a small but significant complication risk (1:1000 of severe bleeding, especially in coagulopathic patients). Furthermore, only 0.002% of the liver is examined, and there is great intra- and inter-observer variability in histological interpretation (see, e.g. "Sampling error and intra-observer variation in liver biopsy in patients with chronic HCV infection". Regev A et al., Am. J. Gastroenterol. 2002 Oct; 97(10):2614-8; Janiec DJ et al., "Histologic variation of grade and stage of non-alcoholic fatty liver disease in liver biopsies", Obes. Surg. 2005 Apr;15(4):497-501; and El Badry AM et al., "Assessment of Hepatic Steatosis by Expert Pathologists: The End of a Gold Standard", Annals of Surgery 250(5), Nov 2009, 691-697).

A relatively high proportion of patients referred for liver biopsy have high liver iron. Fibrosis cannot be assessed accurately in this population using a non-invasive imaging procedure such as T1 mapping without some kind of correction.

A similar issue is raised with patients having variable amounts of fat in their liver and other visceral organs. Liver fat fractions varies in the range 0% to 50% (Tang A et al. "Nonalcoholic Fatty Liver Disease: MR Imaging of Liver Proton Density Fat Fraction to Assess Hepatic Steatosis" Radiology 2013;267:422-431; Idilman IS et al., "Hepatic steatosis: quantification by proton density fat fraction with MR imaging versus liver biopsy" Radiology 2013;267:767-775; and Szczepaniak LS et al., "Magnetic resonance spectroscopy to measure hepatic triglyceride content: prevalence of hepatic steatosis in the general population" Am. J. of Physiol. Endocrinol. Metab. 2005;288:E462-468).

A further issue is that inhomogeneities in the static magnetic field used in magnetic resonance (MR) imaging can lead to off-resonance frequencies that may influence the measurement of the T1.

Accordingly, there is a need to address the aforementioned deficiencies and inadequacies.

Briefly described, methods and systems for processing MR relaxometry data are provided. These may be of value in evaluating fibrosis/inflammation in visceral tissues in the presence of elevated iron and elevated fat, and in the presence of off-resonance frequencies in the MR system.

In magnetic resonance (MR) imaging, tissue contrast is generated by a combination of intrinsic tissue properties such as spin-lattice (T1) and spin-spin (T2) relaxation times, and extrinsic properties such as imaging strategies and settings. Signal intensity in conventional MR images is displayed on an arbitrary scale, and thus is not adequate for direct comparisons. T1 relaxation times depend on the composition of tissues. T1 relaxation times exhibit characteristic ranges of normal values at a selected magnetic field strength. Deviation from established ranges can thus be used to quantify the effects of pathological processes.

In the nineties, some groups identified elevated T1 in the livers of patients with cirrhosis (e.g. Thomsen et al., "Prolonged T1 in patients with liver cirrhosis: An in vivo MRI study. Magn. Reson. Imaging". 1990; 8:599-604; and Keevil et al., "Non-invasive assessment of diffuse liver disease by in vivo measurement of proton nuclear magnetic resonance relaxation times at 0.08 T". Br. J. Radiol. 1994; 67:1084-1087), but this did not gain widespread acceptance, perhaps in part because of conflicting experimental data (Goldberg et al. Hepatic cirrhosis: magnetic resonance imaging. Radiology. 1984; 153:737-9; Chamuleau et al. "Is the magnetic resonance imaging proton spin-lattice relaxation time a reliable noninvasive parameter of developing liver fibrosis?" Hepatology. 1988; 8:217-21; and Aisen et al. "Detection of liver fibrosis with magnetic cross-relaxation". Magn. Reson. Med. 1994; 31 :551-6), and a lack of easily applied in-vivo T1-mapping methods.

More recently, with the development of robust, single breath-hold T1 mapping techniques (e.g. Piechnik et al., "Shortened Modified Look-Locker Inversion recovery (ShMOLLI) for clinical myocardial T1-mapping at 1.5 and 3 T within a 9 heartbeat breathhold". J. Cardiovasc. Magn. Reson. 2010 12:69), interest in T1-mapping of the liver in patients with cirrhosis has increased again (Heye et al. "MR relaxometry of the liver: significant elevation of T1 relaxation time in patients with liver cirrhosis". Eur. Radiol. 2012; 22:1224-32; and Kim et al. "Quantitative evaluation of liver cirrhosis using T1 relaxation time with 3 tesla MRI before and after oxygen inhalation". J. Magn. Reson. Imaging. 2012; 36:405-10).

However, these studies have excluded patients with iron overload. One study (Henninger et al. Evaluation of MR imaging with T1 and T2* mapping for the determination of hepatic iron overload. Eur. Radiol. 2012; 22:2478-86) has addressed the additional information that can be gained by combining T2* and T1 measurements, but only qualitatively (presence/absence of iron overload or fibrosis).

A new method of analysing MR relaxometry data of liver and other visceral tissues has now been found which can reliably show differences in extracellular fluid (ECF) content in the liver and other visceral tissues and thereby allow quantification of the degree of liver fibrosis and thus serve as a biomarker for liver disease and other visceral tissue diseases, even in the presence of high fat and high iron contents.

In particular, a new approach to interpreting magnetic resonance relaxometry (for example T1, fat, B0 and T2* mapping) of liver disease has been developed.

A multi-compartment model of the liver consisting of at least two compartments, and preferably up to five compartments, is provided, with variable amounts of iron and extracellular fluid. Its behaviour under the exact T1 mapping MRI sequence is then simulated and a reconstruction method is used. These simulation results can be used, for example, as a look-up table, or the simulation can be run iteratively on a per-patient basis. Given the measured iron (for example from T2* mapping), measured fat (for example from proton magnetic resonance spectroscopy), off-resonance frequencies and measured T1, the patient's extracellular fluid fraction can then be inferred, thus giving an indication of the level of liver fibrosis/inflammation.

The invention is defined by the computer-implemented method of independent claim 1, the system or apparatus of independent claim 11 and the software-bearing carrier of independent claim 12. The inventive method compares the raw measured and simulated complex bSSFP (balanced Steady-State Free Precession) signals. This guarantees the uniqueness of the matched solution across scanners with respect to data-fitting algorithms, i.e. the matched simulated signal, and, ultimately, the corrected T1 that may be determined based on that match.

The invention provides a computer-implemented method for processing MR relaxometry data of a visceral tissue of a subject.

The visceral tissue may be any internal organ of the subject's body or part or tissue thereof, preferably wherein the organ is a liver, kidney, heart, pancreas or spleen.

In some particularly-preferred embodiments, the visceral tissue is liver or a liver tissue.

The subject may be any animal, preferably a mammal, most preferably a human. In some embodiments, the subject may be one with liver disease, preferably one with liver fibrosis or liver cirrhosis or non-alcoholic fatty liver disease (NAFLD) or non-alcoholic steatohepatitis (NASH). In some embodiments, the subject may be one with liver disease and portal hypertension, preferably one with cirrhosis or liver fibrosis. In some embodiments, the subject may be one with portal hypertension, preferably one with non-cirrhotic portal hypertension or pre-hepatic portal hypertension or hepatic portal hypertension or post-hepatic portal hypertension. In some embodiments, the subject may be one with heart disease, preferably one with right heart failure or congestive heart failure or congenital heart disease or constrictive pericarditis or tricuspid valve disease or valvular heart disease. In some embodiments, the subject may be one with liver disease that is a consequence of heart disease, preferably one with cardiac liver cirrhosis. In some embodiments, the subject may be one with increased central venous pressure.

The measurement of T1 relaxometry data may be obtained using a medical imaging device. The T1 relaxometry data may be representative of extracellular fluid in the subject's visceral tissue.

The medical imaging device may be, for example, a magnetic resonance imaging (MRI) device or magnetic resonance (MR) scanner. A subject, such as a patient, may be positioned in association with the medical imaging device.

In one or more aspects, the MR scanner can be used to measure one or more characteristic relaxation times in the visceral tissue (e.g. liver). For example, the visceral tissue can be measured for extracellular fluid content using an MR scanner.

Medical imaging, for example magnetic resonance imaging (MRI), can be used to measure tissue characteristics that can in combination help to determine the presence and severity of liver disease, including in particular, liver fibrosis. MRI can be a powerful tool in the diagnosis of liver disease. In recent years the use of relaxometry, i.e. the measurement of the characteristic relaxation times in liver tissue, has become more widespread, due to the sensitivity of T2 and T2* to iron accumulation in the liver (e.g. St Pierre, et al. "Noninvasive measurement and imaging of liver iron concentrations using proton magnetic resonance", Blood. 2005; 105:855-61; and Wood, et al. "MRI R2 and R2* mapping accurately estimates hepatic iron concentration in transfusion-dependent thalassemia and sickle cell disease patients", Blood, 2005; 106:1460-5).

Using MR relaxometry to measure one or more characteristic times in the visceral tissue can reliably show differences in extracellular fluid (ECF) content.

Preferably, the visceral tissue is measured for extracellular fluid using T1 mapping.

T1 relaxometry is an MRI technique that attempts to measure the T1 relaxation time. T1 relaxation time is an inherent property of tissues and organs that can be measured using MRI scans. T1 relaxation time increases with increases in extracellular fluid in the organs where it is measured. Extracellular fluid can accumulate in tissues and organs for three main reasons: scarring, inflammation and increased pressure/engorgement of the tissues. When assessing a specific organ, for example the liver or spleen, the T1 relaxation time for each pixel location can be mapped onto a quantitative image, forming a T1 map, the basis of this technique. The resulting T1 can then be corrected for the confounding presence of iron, something that is likely to result in greater accuracy.

In the liver, T1 relaxation time increases with increasing burden of scarring. As more scarring accumulates in the liver, more pressure accumulates in the vessels of the gut, liver and spleen (portal hypertension).

A higher T1 relaxation time which is determined from T1 mapping of the visceral tissue for extracellular fluid measurement is an indication of fibrosis in the visceral tissue. A higher T1 relaxation time can indicate a higher degree of hepatic fibrosis or active hepatitis in the liver.

Any T1 mapping method may be applied for acquiring MR relaxometry measurements or data, as long as the details for the T1 mapping sequence are known. Suitable T1 mapping methods include, but are not limited to the spin-lattice T1 mapping that can be performed using repeated inversion recovery (IR) experiments. For example, a modified Look Locker inversion (MOLLI) recovery pulse sequence can be performed. The MOLLI sequence is generally described in Messroghli DR et al. "Modified Look-Locker inversion recovery (MOLLI) for high resolution T1 mapping of the heart". Magn. Reson. Med. 2004; 52:141-146.

In one or more further embodiments, among others, where a shortened breath-hold is desired, the spin-lattice relaxation time (T1) mapping can be performed using a shortened modified Look Locker inversion recovery (Sh-MOLLI) sequence. The Sh-MOLLI sequence is generally described in Piechnik SK, et al., "Shortened Modified Look-Locker Inversion recovery (ShMOLLI) for clinical myocardial T1-mapping at 1.5 and 3 T within a 9 heartbeat breathhold", J. Cardiovasc. Magn. Reson. 2010 Nov 19;12:69. It can also be applied to the family of saturation recovery T1-mapping methods, or variable flip angle T1-mapping methods.

In any one or more embodiments of the invention, the spin-lattice relaxation time (T1) mapping can be performed using consecutive inversion-recovery (IR) experiments, wherein the consecutive IR experiments comprise a first IR experiment, a second IR experiment, and a third IR experiment, the first IR experiment comprising a number of samples exceeding a number of samples of both the second IR experiment and the third IR experiment. The method further comprises conditionally processing the samples in the first, second, and third IR experiments.

These examples are not exhaustive. T1 mapping could also be carried out using, for example, saturation recovery, multiple-flip-angle, or MR fingerprinting methods.

Preferably, T1 mapping is performed using a modified Look Locker inversion (MOLLI) recovery pulse sequence or a shortened modified Look Locker inversion recovery (Sh-MOLLI) sequence.

Balanced steady-state free precession (bSSFP) is a magnetic resonance imaging sequence that allows fast image acquisition. It has a number of attractive characteristics, e.g. high signal and the somewhat unique T2/T1 contrast, as opposed to the more conventional T1 and T2 contrasts (see Bieri and Scheffler, "Fundamentals of Balanced Steady State Free Precession MRI", JOURNAL OF MAGNETIC RESONANCE IMAGING 38:2-11 (2013)).

Preferably, the bSSFP sequence is used with the MOLLI or (shortened)MOLLI T1 mapping sequences, e.g. to sample the recovery period of the inverted MR signal.

Data acquisition is followed by reconstruction of T1 maps. This may be performed my methods well known in the art. One exemplary method is given as follows: This is done on a voxel-by-voxel basis, where signal from each voxel is fitted to a mono-exponential inversion recovery model function with three parameters: $S(TI) = A - B\exp(-TI/T1^*)$, where S is the measured signal at time points TI (inversion time), A and B are parameters related to inversion efficiency and proton density and $T1^*$ is the apparent T1 time, i.e. the time constant of the exponential recovery (and, implicitly, of the bSSFP signal). This $T1^*$ is then transformed into a T1 value using: $T1 = T1^* (B/A-1)$.

Since in MRI signal detection happens using coils in quadrature (i.e. the MR signal picked up on one coil has a 90° phase shift compared to the signal picked up on the other coil), any measured signal will be complex, as the individual coils provide the real and the imaginary part of the measured signal. The measured bSSFP signal will therefore be a complex bSSFP signal.

A number of bSSFP sequences are known, e.g. TrueFISP, FIESTA and balanced FFE. The presence of fat can alter the measured T1 with Look-Locker methods employing balanced steady-state free precession readouts, e.g. MOLLI or shMOLLI. Risk factors that can cause fat to appear in the liver include obesity, alcohol consumption, type II diabetes, high cholesterol levels and smoking.

Due to the difference in resonant frequencies of the water and the different lipid spectral peaks, lipids bSSFP signals have a different dependence on off-resonance frequencies than water, depending on the repetition time of the readout and the field strength. This difference in signals leads to an overall bSSFP signal that, although a weighted sum of the water and lipid signals, does not exhibit a mono-exponential behaviour anymore, altering the T1 value resulting from fitting it to a mono-exponential curve.

Measuring fat fraction, off-resonance frequencies and optionally also taking the bSSFP repetition time into consideration allows correcting for the changes caused by fat.

Determination of the fat content may be done by any suitable technique.

Examples of suitable techniques include $^1$H MR spectroscopy, multiple-echo spoiled gradient echo methods (e.g. Dixon, IDEAL) water-echo imaging and by biopsy.

Preferably, the fat content of the visceral tissue is determined by $^1$H MR spectroscopy, most preferably by using the stimulated echo acquisition mode (STEAM) sequence.

In Step (b), a measurement for the iron content of the visceral tissue is determined.

It has been discovered that elevated liver iron, or iron overload, can alter the T1 relaxation time and its measurement.

Mild iron overload is relatively common in the general population, and higher still in patients with suspected liver disease. The most important causes of iron overload are hereditary hemochromatosis (HHC), a highly prevalent genetic disease with autosomal dominant heritability, transfusion iron overload, and chronic liver disease. Iron overload tends to lower T1 relaxation time and, through its effects on T2 and T2*, also affect the precision of its measurement using a particular sequence and, thereby, cause the measured T1 relaxation time to underreport, for example, extracellular fluid measurement. Iron overload commonly causes liver cirrhosis if left untreated, so the two commonly coexist.

Measuring iron content allows correcting for under-reporting by T1 values when iron overload is present.

Determination of the iron content in Step (b) may be done by any suitable technique, e.g. MR spectroscopy, T2 mapping and T2* mapping.

The visceral tissue (e.g. liver) can be measured for iron content using MR spectroscopy.

The subject's visceral tissue (e.g. liver) can be measured for iron content using T2 mapping.

T2 mapping may be carried out as set forth in St Pierre et al., Noninvasive measurement and imaging of liver iron concentrations using proton magnetic resonance. Blood. 2005; 105:855-61.

The subject's visceral tissue (e.g. liver) can be measured for iron content using T2* mapping. T2* mapping can determine the degree of iron overload. Iron overload of the liver is toxic and causes fibrosis, and causes a reduced T2* value.

Other methods can be used to measure iron content besides T2 or T2* mapping.

The subject's visceral tissue (e.g. liver) can be measured for iron content by measuring one or more blood biomarkers, such as ferritin, transferrin, transferrin saturation, hepcidin, soluble transferrin receptor (sTfR) index (sTfR / log ferritin), or MR spectroscopy. For example, the width of the $^1$H MRS spectra can indicate higher than normal iron loads.

One method is to measure dry weight iron from a separate liver biopsy: normal liver typically has less than 3 mmols per 100g of liver tissue.

Preferably, the iron content of the visceral tissue is measured using T2* mapping.

A measurement for the off-resonance frequencies of the MR system, e.g. an MR imaging system, may be determined.

It is known that the frequency response of steady-state free-precession (SSFP) sequences often results in dark-band artifacts. Since T1-mapping methods such as MOLLI use SSFP readout, they are therefore prone to frequency-dependent errors in T1-measurements. Such errors may be due to off-resonance frequencies.

Off-resonance frequencies represent deviations from the central frequency of the MRI scanner. They are proportional to the inhomogeneity of the static magnetic field ($B_0$). Generally, the off-resonance frequencies fall in the range -150 Hz to +150Hz (Kellman P. et al., "Influence of Off-resonance in myocardial T1-mapping using SSFP based MOLLI method", J. Cardiovasc. Magn. Reson. 2013 Jul 22;15:63; and Mozes FE, et al., "Influence of fat on liver T1 measurements using modified Look-Locker inversion recovery (MOLLI) methods at 3T", J. Magn. Reson. Imaging. 2016 Jan 13; 44:105-111).

The off-resonance behaviour of SSFP and associated banding artifacts are often analysed assuming that the magnetisation is at steady-state, as in continuous cine imaging. However, the MOLLI imaging sequence uses single-shot imaging with data acquired on the approach to steady-state. As a result, the off-resonance response becomes dependent on the initial condition, which is in turn dependent on the inversion recovery time. This leads to a frequency dependent change in the apparent inversion recovery.

It is now becoming apparent that even small off-resonance frequencies that are well within the region without banding artifacts may result in significant T1 errors. Such errors will become more significant as T1 mapping is used to detect more subtle pathologies.

Determination of the off-resonance frequencies in Step (b) may be done by any suitable technique.

From a practical perspective, it is not possible to take the derivative of the phase, but it is possible to approximate it. For this, two gradient recalled echo (GRE) images may be acquired, e.g. with echo times of 2.46 ms and 4.92 ms, for a 3T field. The formula:

$$f = \frac{1}{2\pi}\frac{\phi_2 - \phi_1}{TE_2 - TE_1}\ [Hz]$$

may then be applied. If echo times are chosen such that water and fat are in phase, these times will be dependent on the field strength of the scanner that is used.

Off-resonance frequencies can be determined from the same data that provides T2* information.

Step (b) may also include determining one or more additional parameters. These additional parameters may include one or more of the following:

(a) TR -The repetition time of the balanced steady-state free precession (bSSFP) readout used by the (sh)MOLLI sequence. Its influence on T1 values is described in [10].
(b) TE - The echo time of the bSSFP readout.
(c) RR interval - The distance between two R peaks of the ECG signal that is monitored during the MRI. RR = 60 / (heart rate) in seconds. ShMOLLI T1 is fairly independent of the heart rate.

According to the invention, the method involves the use of a simulated T1 measurement of the subject's visceral tissue for different fractions of extracellular fluid for the determined fat content, iron content and off-resonance frequencies.

The simulation may include multi-compartment modeling of various fractions of extracellular fluid in the visceral organ (e.g. liver) and fat and the impact of iron content and off-resonance frequencies in the visceral organ (e.g. liver) on both the intra- and extra-cellular relaxation times.

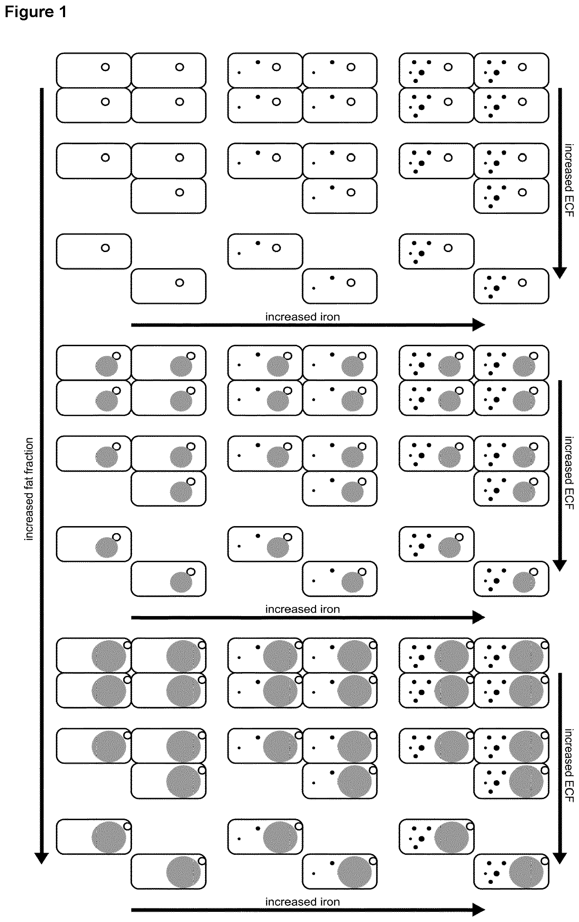

In an example of a three-compartment model, shown in Figure 1, compartments correspond to hepatic lipid content, intra- and extra-cellular fluid, the proportions of which can be varied. The quantity of iron in the cells can also be varied, corresponding to different hepatic iron contents.

The model of the liver is preferably based on a five compartment model. One example of such a model is described in [5] that is extended with a fifth compartment of hepatic fat content.

The five compartment model may comprise an extracellular compartment made up of blood and interstitial fluid, and an intracellular compartment composed of a liquid and a semi-solid pool. The liquid pool represents the "bulk" water freely moving around in the hepatocytes, while the semi-solid pool represents water molecules bound to macromolecules in the hepatocytes; their motion is restricted by the hydrogen bonds connecting them to these macromolecules. Preferably, a distinction is made between the semi-solid and the liquid pool because there is magnetization exchange between them, which is known to decrease shMOLLI T1 [11]. Exchange also occurs between blood and interstitial fluid, but the rate of this exchange is in the limit of fast exchange, i.e. the exchange rate is much faster than the difference between relaxation rates of the two compartments.

Preferably, therefore, the model of the visceral tissue (e.g. liver) is based on a five-compartment model having the following compartments:

(i) an extracellular blood compartment;
(ii) an extracellular interstitial fluid compartment,
(iii) an intracellular liquid pool compartment;
(iv) an intracellular semi-solid pool compartment; and
(v) an intracellular lipid (fat) compartment.

Due to the fast exchange occurring between blood and interstitial fluid, the longitudinal and transverse relaxation rates may be combined taking into consideration volume fractions [5]. The evolution of magnetisation over time may be simulated using the Bloch equations for the extracellular fluid, intracellular liquid compartment and intracellular semi-solid compartment.

The additional fat compartment represents protons of triglycerides concentrated in fat droplets in hepatocytes. Due to the hydrophobic nature of long chains of fat, they do not bind water molecules and thus do not show magnetization transfer effects with the bulk water of the cytosol.

The magnetic resonance behaviour of the water in these compartments may be modeled using knowledge of the field strength of the MR relaxometry and exact details of the T1-mapping pulse sequence, which could be, for example, any of inversion recovery, saturation recovery or variable flip angle T1-mapping method. The model can be, for example, a full Bloch simulation.

The field strength of the scanner may be used to determine the chemical shift difference between the water and other lipid spectral peaks, i.e. the difference between their resonance frequencies.

The simulating step may also incorporate measurements of or for one or more of TR, TE and RR-interval.

The value of the repetition time (TR) and echo time (TE) are generally set during the image acquisition phase; they may be saved in images obtained from the MR scanner. The RR interval may be measured by recording the ECG signal from electrodes on the subject's chest and calculating the distance between two R waves in the ECG trace. This time interval may be recorded for each image of the shMOLLI sequence.

Fat may be modelled as a separate pool of protons in the hepatocytes. For example, six spectral peaks may be simulated for the fat, as described in Hamilton et al. "In vivo characterization of the liver fat 1H MR spectrum" NMR Biomed. 2011; 7:784-790. T1 and T2 may be fixed to values measured by magnetic resonance spectroscopy in an appropriate phantom (e.g. a peanut oil phantom). Some of the T2 values are available from Hamilton et al. "Effect of PRESS and STEAM sequences on magnetic resonance spectroscopic liver fat quantification" J. Magn. Reson. Imaging 2009; 30:145-152. Since fat is concentrated in droplets and is composed of large, slow molecules, the effects of iron on the T1 and T2 are negligible.

The iron correction equations for the simulation may be based on those of Tunnicliffe [5]. These equations describe R1 and R2 dependencies on iron (R1 and R2 relaxation rates which are related to T1 and T2 relaxation times by: R1 = 1/T1 and R2 = 1/T2).

At 1.5T, transverse relaxation rates (in $s^{-1}$) of liver tissue as a function of hepatic iron content (HIC, measured in mg Fe/g dry weight) are given by St Pierre et al. (Noninvasive measurement and imaging of liver iron concentrations using proton magnetic resonance. Blood. 2005; 105:855-61; and Wood et al., MRI R2 and R2* mapping accurately estimates hepatic iron concentration in transfusion-dependent thalassemia and sickle cell disease patients. Blood. 2005; 106:1460-5):

$$R2(1.5T) = 6.88 + 26.06 \times (HIC)^{0.701} - 0.438 \times (HIC)^{1.402} \qquad (1)$$

$$R2^*(1.5T) = (HIC - 0.202) / 0.0254.$$

To convert these to relaxation rates of liver at 3T (Ghugre et al. Multi-field behaviour of Relaxivity in an Iron-rich environment. Proc Intl Soc Mag Reson Med. 2008; 16:644 and Storey P et al. "R2* imaging of transfusional iron burden at 3T and comparison with 1.5T", J. Magn. Reson. Imaging 2007;25:540-547):

$$R2(3T) = R2(1.5T) \times 1.47 - 2.2 \qquad (2)$$

$$R2^*(3T) = 2 \times R2(1.5T) - 11.$$

Equations 1 and 2 refer to intact liver tissue. However, Equation 1 was obtained using a bi-exponential fit, with the reported R2 calculated from a weighted average of the relaxation rates calculated from the two exponentials. Thus R2 may be expressed as:

$$R2(1.5T) = R2_L(1.5T) \times v_L + R2_E(1.5T) \times v_E \qquad (3)$$

Based on the fact that most diseases have relatively mixed iron deposition, it is assumed that iron deposition is homogeneous between the parenchyma and extracellular space, and that Equation 2 holds for both compartments separately.

The R2 of blood at 3T in the absence of iron deposits is 3.64/s, and that of plasma 2.9/s. In considering the effect of iron on the extracellular relaxation rates, there are limited data on the relative diffusivities of water in the hepatic intra- and extracellular spaces. In the main simulation, it is assumed that the diffusivity of protons in the extracellular space is higher than in tissue as a whole and close to that of free water, around four times higher than that of intact liver tissue. Based on the Monte Carlo simulations of Ghugre and Wood, diffusivity close to free water reduces the iron dependence R2 by 30%. Combining these observations with Equations 1 and 2, the following equations are obtained for the R2 of blood ($R2_B$) and interstitium ($R2_I$) at 3T:

$$R2_B = 3.64 + (0.7 \times 26.06 \times (HIC)^{0.701} - 0.7 \times 0.438 \times (HIC)^{1.402}) \times 1.47 \qquad (4)$$

$$R2_I = 2.9 + (0.7 \times 26.06 \times (HIC)^{0.701} - 0.7 \times 0.438 \times (HIC)^{1.402}) \times 1.47 \quad (5)$$

However, as the two compartments are in fast exchange, the relaxation rate of the extracellular compartment can be expressed simply as:

$$R2_E = (R2_B v_B + R2_{IV_I}) / v_E$$

$$= (3.64 v_B + 2.9 v_I) / v_E + 26.82 \times (HIC)^{0.701} - 0.451 \times (HIC)^{1.402} \quad (6)$$

In summary, Equation 7 describes the dependence of transverse relaxation rates on iron in the case of interstitial fluid, blood and intracellular liquid. The generic form of such an equation is:

$$R_2 = R_{20} + a\text{HIC}^{0.710} + b\text{HIC}^{1.402} \quad (7)$$

where $R_2$ is the transverse relaxation rate, $R_{20}$ is the transverse relaxation rate in the absence of iron, $a$ and $b$ are scaling factors that can be determined experimentally, as described in [12].

Equation 8 describes the dependence of longitudinal relaxation rates on iron for interstitial fluid, blood and intracellular liquid. The generic form of such an equation is:

$$R1 = R_{10} + 0.029\text{HIC}, \quad (8)$$

where $R_1$ is the longitudinal relaxation rate, $R_{10}$ is the longitudinal relaxation rate in the absence of iron [13]. Values of $R_{10}$ for the interstitial fluid and blood were taken from [5]. The initial value of intracellular longitudinal relaxation rate was the only free parameter of the simulation and it needed to be set such that for normal iron (1 mg/g dry weight), 0% fat and 0 Hz off-resonance frequency the simulated shMOLLI T1 would be equal to that of normal volunteers, i.e. 717 ms, as reported in [14]. This equation can be used both for a field strength of 3T and 1.5T, due to the low field-sensitivity of ferritin R1 [15] and limited effect of haemosiderin on T1 values [16].

Since magnetic resonance is sensitive to parts per million (ppm) level in deviations in the $B_0$ field, different off-resonance frequencies (e.g. 1 Hz increment between 0 and 640 Hz, 2 Hz between 640 to 680 Hz, 10 Hz between 690 to 6250 Hz, and 20 Hz between 6270 to 6400 Hz) may be simulated for each combination of T1 and T2 parameters to incorporate the effects of signal evolutions in different $B_0$ fields.

The phase accumulation or phase loss caused by off-resonance frequencies may be incorporated in the Bloch equation simulation or the Bloch-McConnell equation simulation.

The simulation step may include simulating a predicted measurement of the visceral organ (e.g. liver) for various fractions of extracellular fluid and the impact of fat content, iron content and off-resonance frequencies on both the intra- and extracellular fluid relaxation times in combination with a simulation of an imaging sequence, for example an imaging sequence involving T1 mapping.

The output of this simulation is the dependence of the measured T1 on the different variables, for example extracellular fluid fraction, fat, iron and off-resonance frequencies. If the iron and fat have been independently measured, for example using T2* mapping in case of the iron and proton MR spectroscopy in case of the fat, it is then possible to assess the extracellular fluid fraction given the hepatic iron content, hepatic lipid content and measured T1, and hence correct the measured T1 for an individual patient, as if the patient's iron and fat levels were normal.

The simulation step may be performed in real time, i.e. as part of the process of the invention, or it may have been previously-performed (e.g. stored in a look-up table).

If the simulation step is performed in real time, the free parameter $R_{10}$ is preferably determined. ($R_{10}$ is the longitudinal relaxation rate in the absence of iron.) This may be done by looping over a range of $R_{10}$ values and simulating a signal for fixed TR, TE and RR values extracted from bSSFP images, normal hepatic iron concentration, 0% fat fraction and on resonance. When the fitted T1 value is equal to the shMOLLI T1 of normal volunteers (i.e. 717 ms), the iteration stops.

The "on-line" correction may only contain one simulation loop iterating over different values of ECF, and every other parameter may be fixed (e.g. TR, TE, RR, off-resonance frequency, hepatic iron concentration and/or fat fraction). The fixed parameters may be taken from the measurement data (e.g. TR, TE and RR may be provided in the shMOLLI bSSFP images; off-resonance frequency and hepatic iron concentration may be determined from the multiple-echo GRE images; fat fraction may be determined from the 1H MRS spectra or multiple-echo GRE images via the Dixon or the IDEAL method). To simulate the effects of iron, the same equations may be used as in the case of building a look-up table.

After an iteration is finished, the simulated signal may be compared to the acquired bSSFP signal. The ECF corresponding to the best matching signal may be designated as the ECF of the examined liver and a final simulation may be performed with the TR, TE and RR values from the bSSFP images, normal hepatic iron concentration (e.g. 1.0 mg/g dry weight), 0 Hz off-resonance frequency, 0% fat fraction and the ECF identified in the previous step. The T1 fitted to the signal obtained this way is the corrected T1 value.

In another embodiment, the simulation step is one which has been previously performed. In this embodiment, a set of simulated signals and fitted T1 values may have been saved in an appropriate computer-accessible format, e.g. in a look-up table or database.

According to the invention, comparing the measurements of the subject's visceral tissue to the simulated measurements of the subject's visceral tissue for extracellular fluid includes comparing the bSSFP signal provided by the readouts of the T1 mapping sequence to simulated bSSFP signals.

In this Step, the determined measurements are compared to the simulated measurements by iterating through different extracellullar fluid (ECF) values. The ECF corresponding to the signal which is most similar to the measured signal will be designated as the ECF of the visceral tissue in question.

According to the invention, determining from said comparison a corrected value of T1 for the subject's visceral tissue based on zero fat content and a normal iron content for the visceral tissue relates to determining from said comparison the extracellular fluid fraction and a corrected value of T1 for the subject's visceral tissue based on zero fat content and a normal iron content for the subject's visceral tissue.

Preferably, the corrected value of T1 for the subject's visceral tissue is based on zero fat content and a normal iron content for the visceral tissue, and 0 Hz off-resonance frequency of the MR system.

For example, a corrected T1 measurement can be determined based on a zero fat content, normal iron content for the liver and 0 Hz off-resonance frequency.

The normal iron content of the liver may be considered as being in the range 0.2-2.0 mg/g dry liver weight (Sirlin C et al. "Magnetic Resonance Imaging Quantification of Liver Iron" Magn. Reson. Imaging Clin. N. Am. 2012;18(3):359).

In some embodiments, the normal iron content of the liver may be taken as 1.0 mg/g dry liver weight.

1.16 mg/g dry weight is the lower normal limit for myocardial iron content according to Wood J, "Impact of Iron Assessment by MRI", AHS Education Book, 2011 Dec 10; 1:443-450.

In some embodiments, the normal iron content of the pancreas may be taken as 1.0-1.2 mg/g dry pancreas weight, preferably 1.0 mg/g dry pancreas weight.

The normal range of iron concentration in the spleen of children is reported in Fischer R et al. ("Assessment of iron stores in children with transfusion siderosis by biomagnetic liver susceptometry", Hematology 1999;60(4):289-299) as being 1.865 mg/g spleen - 3.460 mg/g spleen.

The method may include producing a predicted T1 image or map associated with a particular extracellular fluid fraction of the visceral tissue for zero fat content and normal iron content.

The MR measurements (e.g. the T1, T2 and/or T2* measurements) may be taken in a Region Of Interest (ROI) which may be automatically segmented or chosen by the operator.

For the liver, one or more of the following considerations may be generally made:

(a) The ROI is placed approximately halfway between the porta hepatis and the liver surface in order to avoid interference from the fluid filled structures in the porta hepatis and subcutaneous tissue or air close to the liver surface;
(b) The ROI is placed so as to avoid visible bile ducts and blood vessels; and/or
(c) The ROI is placed in an area that corresponds to good quality images in the T2* map in order to allow T1 and T2* quantification in the same ROI.

If a spectroscopic method is used to measure T1, T2 or T2*, then the ROI can be the average of the voxel where the measurements are taken.

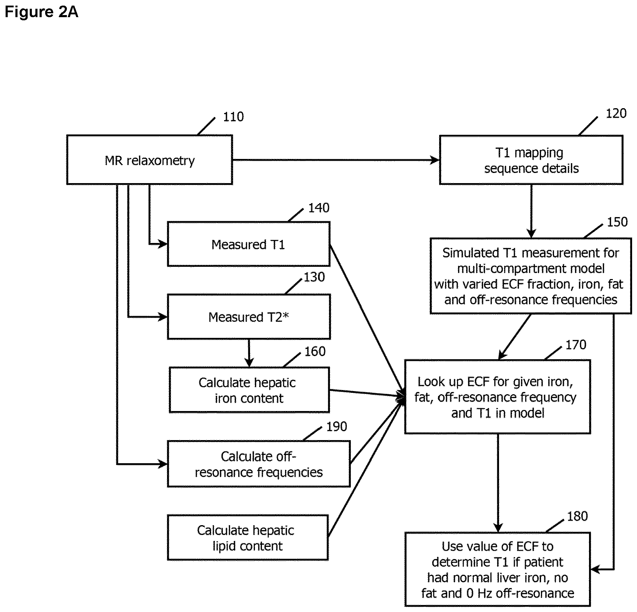

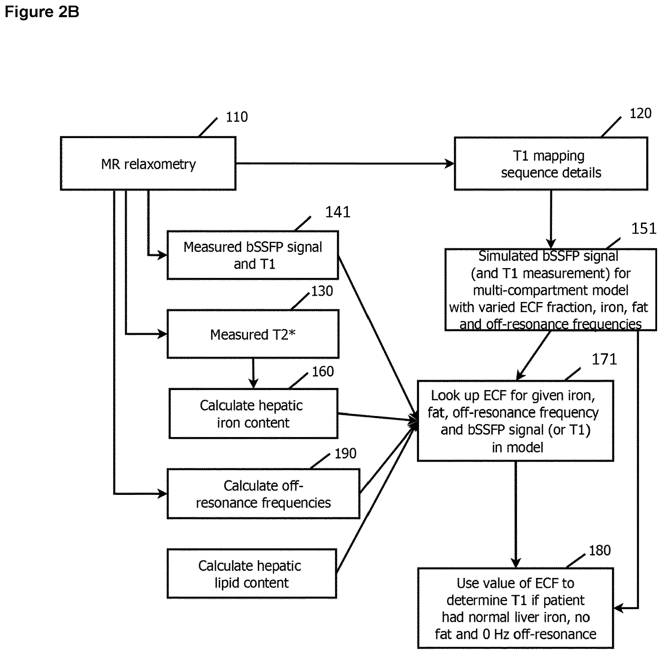

Fig. 2A shows a flow chart of an example method outside the scope of the invention. Fig. 2B shows a flow chart of a method in accordance with the invention. The depicted flowcharts are for performing imaging assessments of a subject's liver, taking into account the presence of fat and iron in the liver and off-resonance frequencies. The simulations apply equally to other visceral organs.

MR relaxometry data (110) which is representative of extracellular fluid is obtained from the subject's liver. The relaxometry data may include T1 (140), T2* (130), off-resonance frequencies (190) and details of the T1 mapping sequence (120).

Off-resonance frequencies can be determined from the same data that provides T2* information.

In these examples, the fat content of the liver may be obtained from 1H MR spectroscopy.

In these examples, the iron content of the liver may be determined (160) from the measured T2*.

In Figure 2A, a measurement (150) of the subject's liver for extracellular fluid (ECF) for a given T1 measurement sequence is simulated. In Figure 2B, a measurement (151) of the subject's liver for extracellular fluid (ECF) for a given bSSFP signal is simulated. For example, a multiple-compartment biophysical model of the microscopic environment of water in the liver is employed. At least two compartments can be adopted: one corresponding to intra-cellular fluid and one to extra-cellular fluid, the proportions of which can be varied.

Preferably, a four-compartment model is used, wherein the model comprises an intracellular semi-solid pool, an intracellular liquid pool, extracellular interstitial fluid and blood.

The quantity of fat and iron in the cells can also be varied in the simulation, and the off-resonance frequencies.

The magnetic resonance behaviour of the water in the compartments can then be modeled using, for example, the relaxation characteristics of water in the different compartments, equations such as Bloch equations, knowledge of the pulse sequence employed to obtain the relaxometry data (110), the method used to calculate the T1 map or bSSFP signal map, and the biophysical model adopted, to simulate a T1 measurement or bSSFP signal.

A Bloch-McConnell simulation [9] may also be used.

The impact of the variable fraction of extra-cellular fluid and fat and iron content and off-resonance frequencies on the relaxation characteristics of water in the different compartments may be determined from published literature and input into the system or method. This allows the determination of the impact of both the variable fraction of extra-cellular fluid and fat and iron content and off-resonance frequencies in the liver on the measured T1 relaxation time or measured bSSFP signal.

The simulated measurements of relaxation time or bSSFP signal for various proportions or fractions of extra-cellular fluid and hepatic iron content and fat content and off-resonance frequencies can then be stored for look-up (170, 171) and comparison to actual relaxation time measurements or bSSFP signals obtained. For example, the measured T1 (140) could be combined with the measured hepatic iron content (160) to find the extracellular fluid fraction used in the simulation which produces that measured T1 in the presence of that iron content. This extracellular fluid fraction can be compared to the normal extra-cellular fluid fraction, for example 25%, to determine the presence of inflammation/fibrosis in the liver. In addition, this value of extra-cellular fluid can be used, for example using the simulated T1 measurement (150), to determine the T1 that would have been measured if the patient's hepatic iron content had been normal (180), to produce an "iron-corrected T1". The measured bSSFP signal and T1 may be similarly processed (Figure 2B, (141)). The simulations may additionally take into account the measured fat content and off-resonance frequencies in a similar manner, in order to produce simulated T1 measurements (Figure 2A, (150)) or simulated bSSFP signals (Figure 2B (151)).

In some embodiments of the invention, the bSSFP signal and T1 are measured (141) and the simulation (151) involves only the bSSFP signal, T1 measurement with varied ECF fractions and iron contents.

The measurement of T1 relaxometry data obtained in Step (a) may be compared with the corrected value of T1 obtained in the method. This information may be useful in determining the patient's level of iron in the visceral organ, the level of fat content in the visceral organ and/or level of off-resonance frequencies in the MR system.

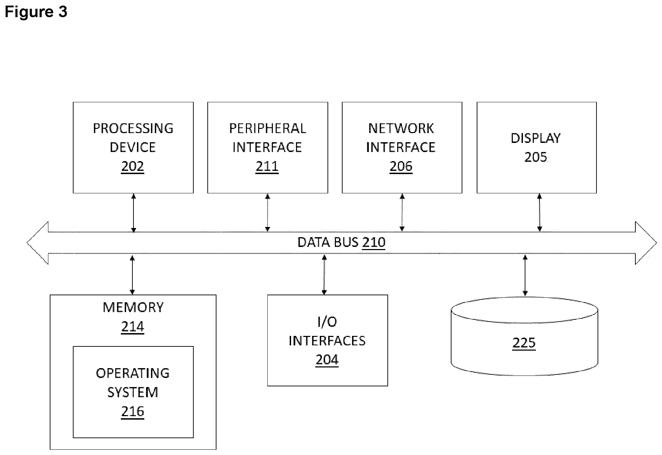

Reference is now made to FIG. 3, which depicts an apparatus in which the systems and methods for evaluating liver fibrosis/inflammation in the presence of elevated iron described herein may be implemented.

The apparatus may be embodied in any one of a wide variety of wired and/or wireless computing devices, multiprocessor computing device, and so forth. As shown in FIG. 3, the apparatus comprises memory (214), a processing device (202), a number of input/output interfaces (204), a network interface (206), a display (205), a peripheral interface (211), and mass storage (225), wherein each of these devices are connected across a local data bus (210). The apparatus may be coupled to one or more peripheral measurement devices (not shown) connected to the apparatus via the peripheral interface (211).

The processing device (202) may include any custom-made or commercially-available processor, a central processing unit (CPU) or an auxiliary processor among several processors associated with the apparatus, a semiconductor based microprocessor (in the form of a microchip), a macro-processor, one or more application specific integrated circuits (ASICs), a plurality of suitably configured digital logic gates, and other well-known electrical configurations comprising discrete elements both individually and in various combinations to coordinate the overall operation of the computing system.

The memory (214) can include any one of a combination of volatile memory elements (e.g., random-access memory (RAM, such as DRAM, and SRAM, etc.)) and nonvolatile memory elements (e.g., ROM, hard drive, tape, CDROM, etc.). The memory (214) typically comprises a native operating system (216), one or more native applications, emulation systems, or emulated applications for any of a variety of operating systems and/or emulated hardware platforms, emulated operating systems, etc. For example, the applications may include application specific software which may be configured to perform some or all of the systems and methods described herein. In accordance with such embodiments, the application specific software is stored in memory (214) and executed by the processing device (202). One of ordinary skill in the art will appreciate that the memory (214) can, and typically will, comprise other components which have been omitted for purposes of brevity.

Input/output interfaces (204) provide any number of interfaces for the input and output of data. For example, where the apparatus comprises a personal computer, these components may interface with one or more user input devices (204). The display (205) may comprise a computer monitor, a screen for a PC, a liquid crystal display (LCD) on a hand held device, or other display device.

In the context of this disclosure, a non-transitory computer-readable medium stores programs for use by or in connection with an instruction execution system, apparatus, or device. More specific examples of a computer-readable medium may include by way of example and without limitation: a portable computer diskette, a random access memory (RAM), a read-only memory (ROM), an erasable programmable read-only memory (EPROM, EEPROM, or Flash memory), and a portable compact disc read-only memory (CDROM) (optical).

With further reference to FIG. 3, network interface device (206) comprises various components used to transmit and/or receive data over a network environment. For example, the network interface (206) may include a device that can communicate with both inputs and outputs, for instance, a modulator/demodulator (*e.g.*, a modem), wireless (e.g. radio frequency (RF)) transceiver, a telephonic interface, a bridge, a router, network card, *etc.*). The apparatus may communicate with one or more computing devices (not shown) via the network interface (206) over a network (118). The apparatus may further comprise mass storage (225). The peripheral (211) interface supports various interfaces including, but not limited to IEEE-1394 High Performance Serial Bus, USB, a serial connection, and a parallel connection.

The apparatus shown in FIG. 3 may be embodied, for example, as a magnetic resonance apparatus, which includes a processing module or logic for performing conditional data processing, and may be implemented either off-line or directly in a magnetic resonance apparatus. For such embodiments, the apparatus may be implemented as a multi-channel, multi-coil system with advanced parallel image processing capabilities, and direct implementation makes it possible to generate immediate T1 maps available for viewing immediately after image acquisition, thereby allowing re-acquisition on-the-spot if necessary. Examples of apparatus in which the T1 mapping sequences, such as the MOLLI and Sh-MOLLI sequences, may be implemented are described in U.S. Patent Nos. 5,993,398 and No. 6,245,027 and U.S. Patent Application Publication No. 2011/0181285.

The flowchart of FIGS. 2A and 2B show examples of functionality that may be implemented in the apparatus of FIG. 3. If embodied in software, each block shown in FIGS. 2A and 2B may represent a module, segment, or portion of code that comprises program instructions to implement the specified logical function(s). The program instructions may be embodied in the form of source code that comprises machine code that comprises numerical instructions recognizable by a suitable execution system such as the processing device (202) (FIG. 3) in a computer system or other system. The machine code may be converted from the source code, etc. If embodied in hardware, each block may represent a circuit or a number of interconnected circuits to implement the specified logical function(s).

Although the flowcharts of FIGS. 2A and 2B show a specific order of execution, it is understood that the order of execution may differ from that which is depicted. For example, the order of execution of two or more blocks may be scrambled relative to the order shown. Also, two or more blocks shown in succession in FIGS. 2A and 2B may be executed concurrently or with partial concurrence. Further, in some embodiments, one or more of the blocks shown in FIGS. 2A and 2B may be skipped or omitted. In addition, any number of counters, state variables, warning semaphores, or messages might be added to the logical flow described herein, for purposes of enhanced utility, accounting, performance measurement, or providing troubleshooting aids, *etc.* It is understood that all such variations are within the scope of the present disclosure.

Also, any logic or application described herein that comprises software or code can be embodied in any non-transitory computer-readable medium for use by or in connection with an instruction execution system such as, for example, a processing device (202) in a computer system or other system. In this sense, each may comprise, for example, statements including instructions and declarations that can be fetched from the computer-readable medium and executed by the instruction execution system.

In a further embodiment, the invention provides a system or apparatus as defined in claim 11. In a further embodiment, the invention provides a carrier bearing software as defined in claim 12.

## BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.

FIG. 1 schematically depicts the changes in the three cellular components of an exemplary model of the current invention: extracellular fluid (ECF) fraction (which increases as cells die), iron overload (iron is primarily stored intracellularly) and fat fraction (fat droplets are stored inside hepatocytes).

Fig. 2A shows a flow chart of an example method outside the scope of the invention. Fig. 2B shows a flow chart of a method in accordance with the invention.

FIG. 3 is a schematic block diagram of an apparatus in which embodiments of the present method disclosed herein may be implemented.

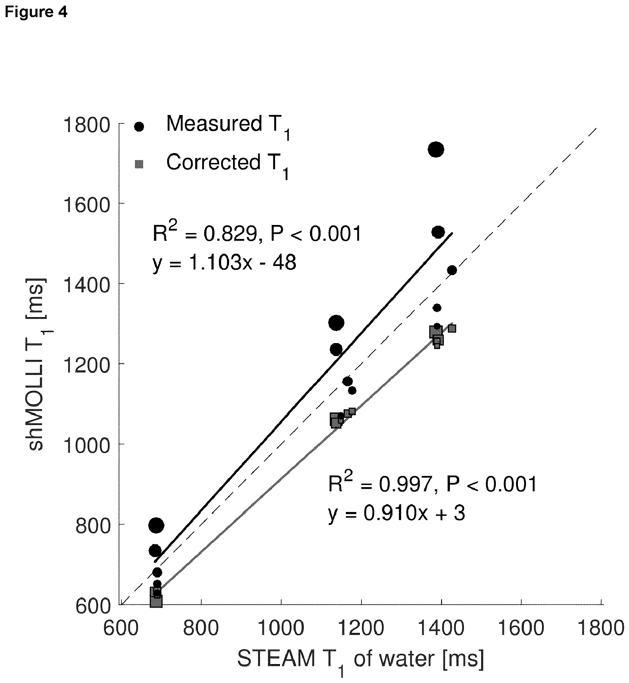

FIG 4: Measured and fat-corrected shMOLLI T1 values in phantoms. The figure shows correlation between spectroscopically measured T1 of the water in phantoms (using STEAM) and shMOLLI T1 of phantoms before and after applying fat correction. Besides the increase in $R^2$, a reduction of the variation of measurements can also be noticed after correction for fat. The dashed line represents the line of identity. Individual symbol sizes are proportional to the amount of fat in each phantom (0%, 5%, 10%, 20% and 30%).

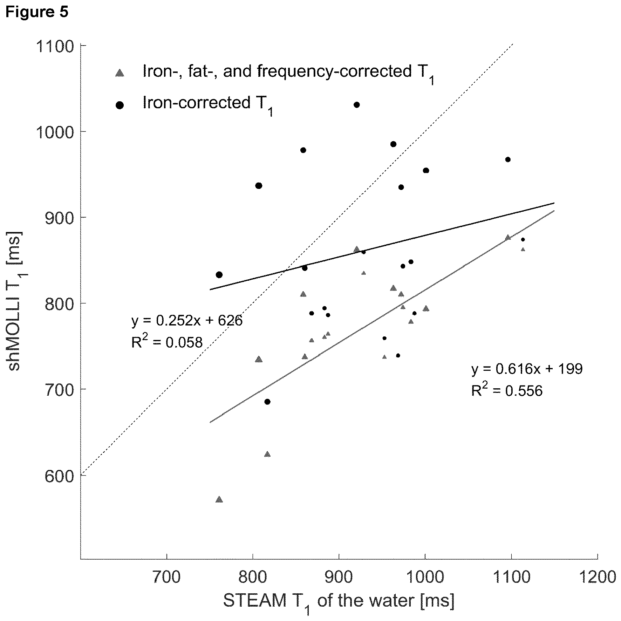

FIG 5: Iron-corrected and fat-, iron- and frequency-corrected shMOLLI T1 values. The figure shows correlation between spectroscopically-measured T1 of the water in the liver of participants and shMOLLI T1 of the liver of participants before and after applying the fat correction. Fat-, iron- and frequency-correction reduces the variability of shMOLLI T1 values and also increases their correlation coefficient with spectroscopically-measured T1 of water in the liver. The dashed line represents the line of identity. Individual symbol sizes are proportional to the amount of fat in each subject's liver (range: 1.5% to 20.3%).

## EXAMPLES

The present invention is further illustrated by the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and can make various changes and modifications of the invention to adapt it to various usages and conditions. Thus, various modifications of the invention in addition to those shown and described herein will be apparent to those skilled in the art from the foregoing description. Such modifications and changes belong to the scope of the invention only if they fall within the scope defined by the claims.

### Example 1: Validation of the MOLLI T1 correction method

### Phantom study

Water and peanut oil emulsions with varying peanut oil concentrations (0%, 5%, 10%, 20% and 30%) were fixed in 2% agar gel, as described in [1]. Three batches of phantoms were built (i.e. a total of 15 phantoms) in order to reflect different T1s of the water component, by adding 0.45 mM, 0.73 mM and 1.61 mM of $NiCl_2$ to the water-oil emulsion.
The phantoms were then scanned using a Siemens 3T Trio Tim imager (Siemens Healthcare, Erlangen, Germany). T2* values and static field inhomogeneities were determined from multiple-echo gradient recalled echo (GRE) images; T1 maps were collected using the shortened modified Look-Locker inversion recovery sequence (ShMOLLI) [2]; and fat fraction was determined from stimulated echo acquisition mode (STEAM) [3] single voxel magnetic resonance spectroscopy (MRS). An additional STEAM MRS sequence involving multiple repetition (TR) and multiple echo times (TR) [4] was used to quantify the T1 and T2 of the water component. The multiple-TR, multiple-TE STEAM sequence was also used to characterise the T1 and T2 values of individual fat peaks in a pure peanut oil phantom.

Phantom model

Phantoms were described using a two-compartment model, comprising a water and a fat compartment. The overall measured balanced steady-state free precession (bSSFP) signal arose as the weighted sum of the individual bSSFP signals from each component. The weighting reflected the fat fraction of the phantom.
The fat component was modelled with six spectral peaks, using the T1 and T2 values determined from the multiple-TR, multiple-TE STEAM MRS sequence. Several water signals were simulated for T1 values in the range of 500-1600 ms. Water T2 values were different for different concentrations of NiCl2 but remained constant between phantoms with different fat fractions at the same $NiCl_2$ concentration. Each water signal was combined with the fat signal in a proportion corresponding to the fat fraction in each phantom. The simulated signal obtained this way was then fitted to the measured bSSFP signal. Equation (1) was used for fitting.

$$Smeas = a \times Ssim \qquad (1)$$

$S_{meas}$ is the measured bSSFP signal, $S_{sim}$ is the simulated signal and a is a fitting parameter. Goodness of fit was evaluated using the $R^2$ coefficient of determination. The water T1 corresponding to the signal with the highest $R^2$ value was then used to simulate a pure water ShMOLLI T1 at 0 Hz off-resonance frequency, equivalent to the ShMOLLI T1 of the phantoms in the ideal case of no fat and no static field inhomogeneities.

**Human participant study**

N = 20 patients (10 females, mean age 52 years) with various levels of hepatic lipid content and hepatic scarring were scanned using the same 3T scanner as mentioned in the phantom study section and the same measurements were performed as in the case of phantoms.

Liver model

The model of the liver described by Tunnicliffe *et al.* [5] was extended with a further tissue compartment representing fat droplets in hepatocytes. Volume fractions of the liquid liver, semi-solid liver and extracellular fluid compartments were adjusted so that they reflected the fat fraction measured by 1H MRS. bSSFP signals corresponding to all fluid compartments and fat were simulated using Bloch equation simulations. The fat signal was modelled using six spectral peaks, as described by Hamilton *et al.* [6]. Due to the similarity between the spectrum of human adipose fat and the spectrum of peanut oil [7], the same individual T1 values were used as they were determined in a peanut oil sample. T2 values for three peaks were taken from the literature [8], while T2 values for the 2.75, 4.2 and 5.3 ppm peaks were considered to be equal to those corresponding to the same peaks of the peanut oil spectrum.

The overall simulated signal was obtained by combining the signals corresponding to fat, liquid liver compartment, semisolid liver compartment and the extracellular fluid compartment. T1 and T2 values of the liver components, other than fat were the same as in [5] and magnetisation transfer effects were considered between the semisolid and liquid compartments of the liver. The extracellular fluid volume fraction (ECVF) was varied between 0.25 and (0.9625 - fat fraction), where 0 corresponds to 0% ECVF, i.e. no extracellular fluid, only cells and 1 corresponds to 100% extracellular fluid and no cells. The ECVF corresponding to the signal which showed the highest level of similarity to the measured signal was used to simulate a signal with 0% fat fraction, no variation of the static field strength and at normal hepatic iron content (HIC) (HIC = 1 mg/g dry weight). Similarity between signals was assessed by the R2 of fitting the simulated signal to the measured signal, as detailed in the phantom study section.

**Results**

In order to assess the validity of the correction, both measured and corrected ShMOLLI T1 values were plotted against water T1 values measured by the multiple-TR, multiple-TE STEAM MRS sequence. It is known that ShMOLLI T1 values underestimate real T1 values (2), therefore a linear relationship was expected to exist between STEAM T1s and corrected T1s, once the variability introduced by off-resonance frequencies and fat were removed.

The same relationship holds for corrected liver T1s as well, since all participants had normal or close to normal iron levels (HIC ≈ 1 mg/g dry weight), and so neither ShMOLLI nor STEAM T1s were reduced by iron. Figure 4 and Figure 5 show results of the correction and results of fitting to STEAM-measured T1 values. An increase in the $R^2$ values, and thus an increase in the strength of the linear relationship, has been observed: 0.829 to 0.997 in case of the phantoms and 0.058 to 0.556 in case of human participants.

**REFERENCES**

1. Hines CDG, Yu H, Shimakawa A, McKenzie CA, Brittain JH, Reeder SB. T1 independent, T2* corrected MRI with accurate spectral modeling for quantification of fat: Validation in a fat-water-SPIO phantom. J Magn Reson Imaging. 2009;30(5):1215-22.

2. Piechnik SK, Ferreira VM, Dall'Armellina E, Cochlin LE, Greiser A, Neubauer S, et al. Shortened Modified Look-Locker Inversion recovery (ShMOLLI) for clinical myocardial T1-mapping at 1.5 and 3 T within a 9 heartbeat breath-hold. J Cardiovasc Magn Reson [Internet]. 2010 Jan [cited 2015 Jan 12];12(1):69.

3. Frahm J, Merboldt K-D, Hänicke W. Localized proton spectroscopy using stimulated echoes. J Magn Reson. Academic Press; 1987;72(3):502-8.

4. Hamilton G, Middleton MS, Hooker JC, Haufe WM, Forbang NI, Allison MA, et al. In vivo breath-hold (1) H MRS simultaneous estimation of liver proton density fat fraction, and T1 and T2 of water and fat, with a multi-TR, multi-TE sequence. J Magn Reson Imaging [Internet]. 2015 Jun 25 [cited 2015 Nov 19];42(6):1538-43.

5. Tunnicliffe EM, Banerjee R, Pavlides M, Neubauer S, Robson MD. A model for hepatic fibrosis: the competing effects of cell loss and iron on shortened modified Look-Locker inversion recovery T 1 (shMOLLI- T 1) in the liver. J Magn Reson Imaging [Internet]. 2016 Jul [cited 2016 Sep 16]

6. Hamilton G, Yokoo T, Bydder M, Cruite I, Schroeder ME, Sirlin CB, et al. In vivo characterization of the liver fat 1H MR spectrum. NMR Biomed. 2011;24(7):784-90.

7. Yu H, McKenzie CA, Shimakawa A, Vu AT, Brau ACS, Beatty PJ, et al. Multiecho reconstruction for simultaneous water-fat decomposition and T2* estimation. J Magn Reson Imaging [Internet]. 2007 Oct [cited 2015 Mar 5];26(4):1153-61.

8. Hamilton G, Middleton MS, Bydder M, Yokoo T, Schwimmer JB, Kono Y, et al. Effect of PRESS and STEAM sequences on magnetic resonance spectroscopic liver fat quantification. J Magn Reson Imaging [Internet]. 2009 Jul [cited 2015 Apr 1];30(1):145-52.

9. McConnell HM, "Reaction rates by nuclear magnetic resonance", J. Chem. Phys. 1958; 28:430-431.

10. Mozes FE, Tunnicliffe EM, Pavlides M, Robson MD. Influence of fat on liver T 1 measurements using modified Look-Locker inversion recovery (MOLLI) methods at 3T. J Magn Reson Imaging [Internet]. 2016 Jul [cited 2016 Aug 30];44(1):105-11.

11. Robson MD, Piechnik SK, Tunnicliffe EM, Neubauer S. T1 measurements in the human myocardium: The effects of magnetization transfer on the SASHA and MOLLI sequences. Magn Reson Med [Internet]. 2013 Jul 15 [cited 2014 Oct 10];670:664-70.

12. Wood JC, Enriquez C, Ghugre N, Tyzka JM, Carson S, Nelson MD, et al. MRI R2 and R2* mapping accurately estimates hepatic iron concentration in transfusion-dependent thalassemia and sickle cell disease patients. Blood [Internet]. 2005 Aug 15 [cited 2015 Feb 11]; 106(4): 1460-5.

13. Ghugre NR, Coates TD, Nelson MD, Wood JC. Mechanisms of tissue-iron relaxivity: nuclear magnetic resonance studies of human liver biopsy specimens. Magn Reson Med [Internet]. 2005 Nov [cited 2015 Feb 9];54(5):1185-93.

14. Banerjee R, Pavlides M, Tunnicliffe EM, Piechnik SK, Sarania N, Philips R, et al. Multiparametric magnetic resonance for the non-invasive diagnosis of liver disease. J Hepatol [Internet]. 2014 Jan [cited 2015 Feb 1];60(1):69-77.

15. Vymazal J, Brooks RA, Zak O, Mcrill C, Shen C, Chiro G Di. T1 and t2 of ferritin at different field strengths: effect on mri. Magn Reson Med [Internet]. 1992 Oct [cited 2015 Mar 19];27(2):368-74.

16. Versluis MJ, Webb AG, van Buchem MA. Detection of cerebral microbleeds: Physical principles, technical aspects and new developments. In: Werring D, editor. Cerebral Microbleeds. Cambridge: Cambridge University Press; 2011. p. 13-21.

## Claims

1. A computer-implemented method for processing magnetic resonance (MR) relaxometry data of a visceral tissue of a subject, comprising:

   a) obtaining a measurement of T1 relaxometry data of a subject's visceral tissue for extracellular fluid from a bSSFP signal measured by a T1 mapping method using a MR system having a static magnetic field;

b) determining by means of measurements fat content of the subject's visceral tissue, iron content of the subject's visceral tissue and off-resonance frequencies of the MR system, wherein the off-resonance frequencies of the MR system represent deviations from the central frequency of the MR system and are proportional to the inhomogeneity of the static magnetic field of the MR system;

c) comparing the bSSFP signal from the T1 mapping method of the subject's visceral tissue of Step (a) to simulated bSSFP signals of the subject's visceral tissue for different fractions of extracellular fluid for the determined fat content, iron content and off-resonance frequencies, the simulated bSSFP signals having been obtained by simulating the T1 mapping method; and

d) determining a corrected value of T1 from the T1 mapping method simulated for zero fat content and a normal iron content, and preferably for zero off-resonance frequency, for the subject's visceral tissue for the fraction of extracellular fluid corresponding to the simulated bSSFP signal having the highest level of similarity among the simulated bSSFP signals to the measured bSSFP signal.

2. The method of claim 1, the method additionally comprising the step of:
simulating bSSFP signals from the T1 mapping method of the subject's visceral tissue for different fractions of extracellular fluid for the determined fat content, iron content and off-resonance frequencies.

3. The method of any one of the preceding claims, wherein the MR relaxometry data is obtained by use of a medical imaging device including a magnetic resonance (MR) scanner and wherein the device is used to measure one or more characteristic relaxation times in a tissue in the visceral tissue.

4. The method of any one of claims 1-3, wherein the T1 mapping is performed using a modified Look Locker inversion (MOLLI) recovery pulse sequence or a shortened modified Look Locker inversion recovery (Sh-MOLLI) sequence.

5. The method of any one of claims 1 to 4, wherein the measurement for fat content of the subject's visceral tissue is obtained by $^1$H MR spectroscopy.

6. The method of any one of the preceding claims, wherein the visceral tissue is measured for iron content using one or more of T2 mapping, T2* mapping, magnetic resonance spectroscopy, or measuring one or more blood biomarkers.

7. The method of any one of claims 1, 2 or 5, wherein the simulation includes the impact of fat content, iron content and off-resonance frequencies in the visceral tissue on both the intra- and extracellular relaxation times in a multi-compartment model of various fractions of extracellular fluid in the visceral tissue,
preferably wherein the multi-compartment model has the following compartments:

(i) an extracellular blood compartment;
(ii) an extracellular interstitial fluid compartment,
(iii) an intracellular liquid pool compartment;
(iv) an intracellular semi-solid pool compartment; and
(v) an intracellular lipid (fat) compartment.

8. The method of any one of claims 1, 2, 5 or 7, wherein the simulation includes the impact of fat content, iron content and off-resonance frequencies on both the intra- and extracellular fluid relaxation times and simulating a predicted measurement of the visceral tissue for various fractions of extracellular fluid in combination with a simulation of an imaging sequence.

9. The method of any one of the preceding claims, wherein the simulation involves a Bloch equation simulation, with or without exchange between intra- and extra-cellular fluid compartments, and with or without magnetisation transfer effects.

10. The method of any one of claims 1 to 9, wherein the visceral tissue is liver, kidney, spleen or heart, preferably liver.

11. A system or apparatus comprising an MR system and at least one computing device and at least one application executable in the at least one computing device, the at least one application comprising logic configured to cause the computing device and the MR system to perform the method of any one of claims 1-10.

12. A carrier bearing software comprising instructions for configuring an MR system and a processor to carry out the steps of the method of any one of claims 1 to 10.

**Patentansprüche**

1. Computerimplementiertes Verfahren zur Verarbeitung von Magnetresonanz- (MR- ) Relaxometriedaten eines viszeralen Gewebes eines Subjekts, umfassend:

   a) Erhalten einer Messung von T1-Relaxometriedaten des viszeralen Gewebes eines Subjekts für extrazelluläre Flüssigkeit aus einem bSSFP-Signal, das durch ein T1-Mapping-Verfahren unter Verwendung eines MR-Systems gemessen wird, das ein statisches Magnetfeld aufweist;
   b) Bestimmen mittels Messungen eines Fettgehalts des viszeralen Gewebes des Subjekts, eines Eisengehalts des viszeralen Gewebes des Subjekts und von Frequenzen außerhalb der Resonanz des MR-Systems, wobei die Frequenzen außerhalb der Resonanz des MR-Systems Abweichungen von der Zentralfrequenz des MR-Systems darstellen und proportional zur Inhomogenität des statischen Magnetfelds des MR-Systems sind;
   c) Vergleichen des bSSFP-Signals aus dem T1-Mapping-Verfahren des viszeralen Gewebes des Subjekts von Schritt (a) mit simulierten bSSFP-Signalen des viszeralen Gewebes des Subjekts für verschiedene Anteile extrazellulärer Flüssigkeit auf den bestimmten Fettgehalt, Eisengehalt und auf Frequenzen außerhalb der Resonanz, wobei die simulierten bSSFP-Signale durch Simulation des T1-Mapping-Verfahrens erhalten wurden; und
   d) Bestimmen eines korrigierten T1-Werts aus dem T1-Mapping-Verfahren, simuliert für einen Fettgehalt von Null und einen normalen Eisengehalt und bevorzugt für eine Frequenz außerhalb der Resonanz von Null, für das viszerale Gewebe des Subjekts für den Anteil der extrazellulären Flüssigkeit, der dem simulierten bSSFP-Signal entspricht, das den höchsten Ähnlichkeitsgrad unter den simulierten bSSFP-Signalen mit dem gemessenen bSSFP-Signal aufweist.

2. Verfahren nach Anspruch 1, wobei das Verfahren zusätzlich den folgenden Schritt umfasst: Simulieren von bSSFP-Signalen aus dem T1- Mapping-Verfahren des viszeralen Gewebes des Subjekts für verschiedene Anteile extrazellulärer Flüssigkeit für den bestimmten Fettgehalt, Eisengehalt und Frequenzen außerhalb der Resonanz.

3. Verfahren nach einem der vorstehenden Ansprüche, wobei die MR-Relaxometriedaten durch Verwendung einer medizinischen Bildgebungsvorrichtung erhalten werden, die einen Magnetresonanz- (MR-) Scanner beinhaltet, und wobei die Vorrichtung zum Messen einer oder mehrerer charakteristischer Relaxationszeiten in einem Gewebe in dem viszeralen Gewebe verwendet wird.

4. Verfahren nach einem der Ansprüche 1-3, wobei das T1-Mapping unter Verwendung einer Modified Look-Locker Inversion Recovery- (MOLLI-) Pulssequenz oder einer Shortend Modified Look-Locker Inversion Recovery- (Sh-MOLLI-) Sequenz durchgeführt wird.

5. Verfahren nach einem der Ansprüche 1 bis 4, wobei die Messung des Fettgehalts des viszeralen Gewebes des Subjekts durch $^1$H-MR-Spektroskopie erhalten wird.

6. Verfahren nach einem der vorstehenden Ansprüche, wobei der Eisengehalt des viszeralen Gewebes unter Verwendung eines oder mehrerer von T2-Mapping, T2*-Mapping, Magnetresonanzspektroskopie oder Messung eines oder mehrerer Blutbiomarker gemessen wird.

7. Verfahren nach einem der Ansprüche 1, 2 oder 5, wobei die Simulation den Einfluss von Fettgehalt, Eisengehalt und Frequenzen außerhalb der Resonanz im viszeralen Gewebe sowohl auf die intra- als auch extrazellulären Relaxationszeiten in einem Mehrkompartimentmodell von verschiedenen Anteilen extrazellulärer Flüssigkeit im viszeralen Gewebe beinhaltet, wobei das Mehrkompartimentmodell bevorzugt die folgenden Kompartimente aufweist:

   (i) ein extrazelluläres Blutkompartiment;
   (ii) ein extrazelluläres interstitielles Flüssigkeitskompartiment,
   (iii) ein intrazelluläres Flüssigkeitspoolkompartiment;
   (iv) ein intrazelluläres halbfestes Poolkompartiment; und
   (v) ein intrazelluläres Lipid- (Fett-) Kompartiment.

8. Verfahren nach einem der Ansprüche 1, 2, 5 oder 7, wobei die Simulation den Einfluss des Fettgehalts, des Eisengehalts und der Frequenzen außerhalb der Resonanz auf die Relaxationszeiten sowohl der intra- als auch extrazellulären Flüssigkeit beinhaltet und eine vorhergesagte Messung der viszeralen Flüssigkeit für das viszerale Gewebe für verschiedene Anteile extrazellulärer Flüssigkeit in Kombination mit einer Simulation einer Bildsequenz simuliert.

9. Verfahren nach einem der vorstehenden Ansprüche, wobei die Simulation eine Bloch-Gleichungssimulation mit oder ohne Austausch zwischen intra- und extrazellulären Flüssigkeitskompartimenten und mit oder ohne Magnetisierungstransfereffekte einbezieht.

10. Verfahren nach einem der Ansprüche 1 bis 9, wobei das viszerale Gewebe Leber, Niere, Milz oder Herz, bevorzugt Leber, ist.

11. System oder Einrichtung, umfassend ein MR-System und mindestens eine Computervorrichtung und mindestens eine Anwendung, die auf der mindestens einen Computervorrichtung ausführbar ist, wobei die mindestens eine Anwendung Logik umfasst, die so konfiguriert ist, dass sie die Computervorrichtung und das MR-System dazu veranlasst, das Verfahren nach einem der Ansprüche 1-10 durchzuführen.

12. Trägerlagersoftware, die Anweisungen zum Konfigurieren eines MR-Systems und eines Prozessor zum Ausführen der Schritte des Verfahrens nach einem der Ansprüche 1 bis 10 umfasst.


**Revendications**

1. Procédé mis en oeuvre par ordinateur pour traiter des données de relaxométrie par résonance magnétique (RM) d'un tissu viscéral d'un sujet, comprenant :

    a) l'obtention d'une mesure de données de relaxométrie T1 du tissu viscéral d'un sujet pour un liquide extracellulaire à partir d'un signal bSSFP mesuré par un procédé de mappage T1 en utilisant un système RM présentant un champ magnétique statique ;
    b) la détermination, au moyen de mesures, de la teneur en graisse du tissu viscéral du sujet, de la teneur en fer du tissu viscéral du sujet et des fréquences hors résonance du système RM, dans lequel les fréquences hors résonance du système RM représentent des écarts par rapport à la fréquence centrale du système RM et sont proportionnelles à l'inhomogénéité du champ magnétique statique du système RM ;
    c) la comparaison du signal bSSFP issu du procédé de mappage T1 du tissu viscéral du sujet de l'étape (a) avec des signaux bSSFP simulés du tissu viscéral du sujet pour différentes fractions de liquide extracellulaire pour la teneur en graisse, la teneur en fer et les fréquences hors résonance déterminées, les signaux bSSFP simulés ayant été obtenus en simulant le procédé de mappage T1 ; et
    d) la détermination d'une valeur corrigée de T1 à partir du procédé de mappage T1 simulé pour une teneur en graisse nulle et une teneur en fer normale, et de préférence pour une fréquence hors résonance nulle, pour le tissu viscéral du sujet pour la fraction de liquide extracellulaire correspondant au signal bSSFP simulé présentant le niveau de similarité le plus élevé parmi les signaux bSSFP simulés avec le signal bSSFP mesuré.

2. Procédé selon la revendication 1, le procédé comprenant en outre l'étape de : simulation des signaux bSSFP provenant du procédé de mappage T1 du tissu viscéral du sujet pour différentes fractions de liquide extracellulaire pour la teneur en graisse, la teneur en fer et les fréquences hors résonance déterminées.

3. Procédé selon l'une quelconque des revendications précédentes, dans lequel les données de relaxométrie RM sont obtenues en utilisant un dispositif d'imagerie médicale incluant un scanner à résonance magnétique (RM) et dans lequel le dispositif est utilisé pour mesurer un ou plusieurs temps de relaxation caractéristiques dans un tissu dans le tissu viscéral.

4. Procédé selon l'une quelconque des revendications 1-3, dans lequel le mappage T1 est effectué en utilisant une séquence d'impulsions de récupération d'inversion de Look Locker modifiée (MOLLI) ou une séquence de récupération d'inversion de Look Locker modifiée raccourcie (Sh-MOLLI).

5. Procédé selon l'une quelconque des revendications 1 à 4, dans lequel la mesure de la teneur en graisse du tissu viscéral du sujet est obtenue par spectroscopie RM [1]H.

6. Procédé selon l'une quelconque des revendications précédentes, dans lequel la teneur en fer du tissu viscéral est mesurée en utilisant un ou plusieurs parmi le mappage T2, le mappage T2*, la spectroscopie par résonance magnétique, ou en mesurant un ou plusieurs biomarqueurs sanguins.

7. Procédé selon l'une quelconque des revendications 1, 2 ou 5, dans lequel la simulation inclut l'impact de la teneur en graisse, de la teneur en fer et des fréquences hors résonance dans le tissu viscéral à la fois sur les temps de relaxation intracellulaire et extracellulaire dans un modèle multi-compartiments de diverses fractions de liquide extracellulaire dans le tissu viscéral, de préférence dans lequel le modèle multi-compartiments présente les compartiments suivants :

(i) un compartiment sanguin extracellulaire ;
(ii) un compartiment de liquide interstitiel extracellulaire ;
(iii) un compartiment de masse liquide intracellulaire ;
(iv) un compartiment de masse semi-solide intracellulaire ; et
(v) un compartiment lipidique (graisse) intracellulaire.

8. Procédé selon l'une quelconque des revendications 1, 2, 5 ou 7, dans lequel la simulation inclut l'impact de la teneur en graisse, de la teneur en fer et des fréquences hors résonance à la fois sur les temps de relaxation de liquide intracellulaire et extracellulaire et la simulation d'une mesure prédite du tissu viscéral pour diverses fractions de liquide extracellulaire en combinaison avec une simulation d'une séquence d'imagerie.

9. Procédé selon l'une quelconque des revendications précédentes, dans lequel la simulation implique une simulation d'équation de Bloch, avec ou sans échange entre compartiments de liquide intracellulaire et extracellulaire, et avec ou sans effets de transfert d'aimantation.

10. Procédé selon l'une quelconque des revendications 1 à 9, dans lequel le tissu viscéral est le foie, le rein, la rate ou le coeur, de préférence le foie.

11. Système ou appareil comprenant un système RM et au moins un dispositif informatique et au moins une application exécutable dans le au moins un dispositif informatique, la au moins une application comprenant une logique configurée pour amener le dispositif informatique et le système RM à effectuer le procédé selon l'une quelconque des revendications 1-10.

12. Support informatique de logiciel comprenant des instructions pour configurer un système RM et un processeur pour exécuter les étapes du procédé selon l'une quelconque des revendications 1 à 10.

Figure 1

**Figure 2A**

**Figure 2B**

**Figure 3**

| PROCESSING DEVICE 202 | PERIPHERAL INTERFACE 211 | NETWORK INTERFACE 206 | DISPLAY 205 |

DATA BUS 210

| MEMORY 214 | I/O INTERFACES 204 | 225 |

OPERATING SYSTEM 216

**Figure 4**

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5993398 A
- US 6245027 B
- US 20110181285

### Non-patent literature cited in the description

- **REGEV A et al.** Sampling error and intra-observer variation in liver biopsy in patients with chronic HCV infection. *Am. J. Gastroenterol.,* October 2002, vol. 97 (10), 2614-8
- **JANIEC DJ et al.** Histologic variation of grade and stage of non-alcoholic fatty liver disease in liver biopsies. *Obes. Surg.,* April 2005, vol. 15 (4), 497-501
- **EL BADRY AM et al.** Assessment of Hepatic Steatosis by Expert Pathologists: The End of a Gold Standard. *Annals of Surgery,* November 2009, vol. 250 (5), 691-697
- **TANG A et al.** Nonalcoholic Fatty Liver Disease: MR Imaging of Liver Proton Density Fat Fraction to Assess Hepatic Steatosis. *Radiology,* 2013, vol. 267, 422-431
- **IDILMAN IS et al.** Hepatic steatosis: quantification by proton density fat fraction with MR imaging versus liver biopsy. *Radiology,* 2013, vol. 267, 767-775
- **SZCZEPANIAK LS et al.** Magnetic resonance spectroscopy to measure hepatic triglyceride content: prevalence of hepatic steatosis in the general population. *Am. J. of Physiol. Endocrinol. Metab.,* 2005, vol. 288, E462-468
- **THOMSEN et al.** Prolonged T1 in patients with liver cirrhosis: An in vivo MRI study. *Magn. Reson. Imaging,* 1990, vol. 8, 599-604
- **KEEVIL et al.** Non-invasive assessment of diffuse liver disease by in vivo measurement of proton nuclear magnetic resonance relaxation times at 0.08 T. *Br. J. Radiol.,* 1994, vol. 67, 1084-1087
- **GOLDBERG et al.** Hepatic cirrhosis: magnetic resonance imaging. *Radiology.,* 1984, vol. 153, 737-9
- **CHAMULEAU et al.** Is the magnetic resonance imaging proton spin-lattice relaxation time a reliable noninvasive parameter of developing liver fibrosis?. *Hepatology.,* 1988, vol. 8, 217-21
- **AISEN et al.** Detection of liver fibrosis with magnetic cross-relaxation. *Magn. Reson. Med.,* 1994, vol. 31, 551-6
- **PIECHNIK et al.** Shortened Modified Look-Locker Inversion recovery (ShMOLLI) for clinical myocardial T1-mapping at 1.5 and 3 T within a 9 heartbeat breathhold. *J. Cardiovasc. Magn. Reson.,* 2010, vol. 12, 69
- **HEYE et al.** MR relaxometry of the liver: significant elevation of T1 relaxation time in patients with liver cirrhosis. *Eur. Radiol.,* 2012, vol. 22, 1224-32
- **KIM et al.** Quantitative evaluation of liver cirrhosis using T1 relaxation time with 3 tesla MRI before and after oxygen inhalation. *J. Magn. Reson. Imaging.,* 2012, vol. 36, 405-10
- **HENNINGER et al.** Evaluation of MR imaging with T1 and T2* mapping for the determination of hepatic iron overload. *Eur. Radiol.,* 2012, vol. 22, 2478-86
- **ST PIERRE et al.** Noninvasive measurement and imaging of liver iron concentrations using proton magnetic resonance. *Blood.,* 2005, vol. 105, 855-61
- **WOOD et al.** MRI R2 and R2* mapping accurately estimates hepatic iron concentration in transfusion-dependent thalassemia and sickle cell disease patients. *Blood,* 2005, vol. 106, 1460-5
- **MESSROGHLI DR et al.** Modified Look-Locker inversion recovery (MOLLI) for high resolution T1 mapping of the heart. *Magn. Reson. Med.,* 2004, vol. 52, 141-146
- **PIECHNIK SK et al.** Shortened Modified Look-Locker Inversion recovery (ShMOLLI) for clinical myocardial T1-mapping at 1.5 and 3 T within a 9 heartbeat breathhold. *J. Cardiovasc. Magn. Reson.,* 19 November 2010, vol. 12, 69
- **BIERI ; SCHEFFLER.** Fundamentals of Balanced Steady State Free Precession MRI. *JOURNAL OF MAGNETIC RESONANCE IMAGING,* 2013, vol. 38, 2-11
- **KELLMAN P. ; 15 et al.** Influence of Off-resonance in myocardial T1-mapping using SSFP based MOLLI method. *J. Cardiovasc. Magn. Reson.,* 22 July 2013, 63

- **MOZES FE et al.** Influence of fat on liver T1 measurements using modified Look-Locker inversion recovery (MOLLI) methods at 3T. *J. Magn. Reson. Imaging.*, 13 January 2016, vol. 44, 105-111
- **HAMILTON et al.** In vivo characterization of the liver fat H MR spectrum. *NMR Biomed.*, 2011, vol. 7, 784-790
- **HAMILTON et al.** Effect of PRESS and STEAM sequences on magnetic resonance spectroscopic liver fat quantification. *J. Magn. Reson. Imaging,* 2009, vol. 30, 145-152
- **ST PIERRE et al.** Noninvasive measurement and imaging of liver iron concentrations using proton magnetic resonance. *Blood.,* 2005, vol. 105, 855-61
- **WOOD et al.** MRI R2 and R2* mapping accurately estimates hepatic iron concentration in transfusion-dependent thalassemia and sickle cell disease patients. *Blood.,* 2005, vol. 106, 1460-5
- **GHUGRE et al.** Multi-field behaviour of Relaxivity in an Iron-rich environment. *Proc Intl Soc Mag Reson Med.,* 2008, vol. 16, 644
- **STOREY P et al.** R2* imaging of transfusional iron burden at 3T and comparison with 1.5T. *J. Magn. Reson. Imaging,* 2007, vol. 25, 540-547
- **SIRLIN C et al.** Magnetic Resonance Imaging Quantification of Liver Iron. *Magn. Reson. Imaging Clin. N. Am.,* 2012, vol. 18 (3), 359
- Impact of Iron Assessment by MRI. **WOOD J.** AHS Education Book. 10 December 2011, vol. 1, 443-450
- **FISCHER R et al.** Assessment of iron stores in children with transfusion siderosis by biomagnetic liver susceptometry. *Hematology,* 1999, vol. 60 (4), 289-299
- **HINES CDG ; YU H ; SHIMAKAWA A ; MCKENZIE CA ; BRITTAIN JH ; REEDER SB.** T1 independent, T2* corrected MRI with accurate spectral modeling for quantification of fat: Validation in a fat-water-SPIO phantom. *J Magn Reson Imaging.,* 2009, vol. 30 (5), 1215-22
- **PIECHNIK SK ; FERREIRA VM ; DALL'ARMELLINA E ; COCHLIN LE ; GREISER A ; NEUBAUER S et al.** Shortened Modified Look-Locker Inversion recovery (ShMOLLI) for clinical myocardial T1-mapping at 1.5 and 3 T within a 9 heartbeat breathhold. *J Cardiovasc Magn Reson,* January 2010, vol. 12 (1), 69
- Localized proton spectroscopy using stimulated echoes. **FRAHM J ; MERBOLDT K-D ; HÄNICKE W.** J Magn Reson. Academic Press, 1987, vol. 72, 502-8
- **HAMILTON G ; MIDDLETON MS ; HOOKER JC ; HAUFE WM ; FORBANG NI ; ALLISON MA et al.** In vivo breath-hold (1) H MRS simultaneous estimation of liver proton density fat fraction, and T1 and T2 of water and fat, with a multi-TR, multi-TE sequence. *J Magn Reson Imaging,* 25 June 2015, vol. 42 (6), 1538-43
- **TUNNICLIFFE EM ; BANERJEE R ; PAVLIDES M ; NEUBAUER S ; ROBSON MD.** A model for hepatic fibrosis: the competing effects of cell loss and iron on shortened modified Look-Locker inversion recovery T 1 (shMOLLI- T 1) in the liver. *J Magn Reson Imaging,* July 2016
- **HAMILTON G ; YOKOO T ; BYDDER M ; CRUITE I ; SCHROEDER ME ; SIRLIN CB et al.** In vivo characterization of the liver fat 1H MR spectrum. *NMR Biomed.,* 2011, vol. 24 (7), 784-90
- **YU H ; MCKENZIE CA ; SHIMAKAWA A ; VU AT ; BRAU ACS ; BEATTY PJ et al.** Multiecho reconstruction for simultaneous water-fat decomposition and T2* estimation. *J Magn Reson Imaging,* October 2007, vol. 26 (4), 1153-61
- **HAMILTON G ; MIDDLETON MS ; BYDDER M ; YOKOO T ; SCHWIMMER JB ; KONO Y et al.** Effect of PRESS and STEAM sequences on magnetic resonance spectroscopic liver fat quantification. *J Magn Reson Imaging,* July 2009, vol. 30 (1), 145-52
- **MCCONNELL HM.** Reaction rates by nuclear magnetic resonance. *J. Chem. Phys.,* 1958, vol. 28, 430-431
- **MOZES FE ; TUNNICLIFFE EM ; PAVLIDES M ; ROBSON MD.** Influence of fat on liver T 1 measurements using modified Look-Locker inversion recovery (MOLLI) methods at 3T. *J Magn Reson Imaging,* July 2016, vol. 44 (1), 105-11
- **ROBSON MD ; PIECHNIK SK ; TUNNICLIFFE EM ; NEUBAUER S.** T1 measurements in the human myocardium: The effects of magnetization transfer on the SASHA and MOLLI sequences. *Magn Reson Med,* 15 July 2013, vol. 670, 664-70
- **WOOD JC ; ENRIQUEZ C ; GHUGRE N ; TYZKA JM ; CARSON S ; NELSON MD et al.** MRI R2 and R2* mapping accurately estimates hepatic iron concentration in transfusion-dependent thalassemia and sickle cell disease patients. *Blood,* 15 August 2005, vol. 106 (4), 1460-5
- **GHUGRE NR ; COATES TD ; NELSON MD ; WOOD JC.** Mechanisms of tissue-iron relaxivity: nuclear magnetic resonance studies of human liver biopsy specimens. *Magn Reson Med,* November 2005, vol. 54 (5), 1185-93
- **BANERJEE R ; PAVLIDES M ; TUNNICLIFFE EM ; PIECHNIK SK ; SARANIA N ; PHILIPS R et al.** Multiparametric magnetic resonance for the non-invasive diagnosis of liver disease. *J Hepatol,* January 2014, vol. 60 (1), 69-77

- **VYMAZAL J ; BROOKS RA ; ZAK O ; MCRILL C ; SHEN C ; CHIRO G DI.** T1 and t2 of ferritin at different field strengths: effect on mri. *Magn Reson Med,* October 1992, vol. 27 (2), 368-74

- Detection of cerebral microbleeds: Physical principles, technical aspects and new developments. **VERSLUIS MJ ; WEBB AG ; VAN BUCHEM MA.** Cerebral Microbleeds. Cambridge University Press, 2011, 13-21